(19) Europäisches Patentamt / European Patent Office / Office européen des brevets

(11) **EP 2 412 401 B1**

(12) **FASCICULE DE BREVET EUROPEEN**

(45) Date de publication et mention de la délivrance du brevet:
**05.01.2022 Bulletin 2022/01**

(51) Int Cl.:
*A61N 1/365* *(2006.01)*   *A61N 1/37* *(2006.01)*
*G06K 9/00* *(2006.01)*

(21) Numéro de dépôt: **11166811.7**

(22) Date de dépôt: **19.05.2011**

(54) **DISPOSITIF MÉDICAL IMPLANTABLE ACTIF COMPRENANT DES MOYENS DE TEST DE CAPTURE VENTRICULAIRE PAR ANALYSE D'UN SIGNAL D'ACCÉLÉRATION ENDOCARDIAQUE**

AKTIVES IMPLANTIERBARES MEDIZINISCHES GERÄT MIT MITTELN ZUM VENTRIKULÄREN EINFANGTESTEN DURCH AUSWERTUNG EINES ENDOKARDIALEN BESCHLEUNIGUNGSSIGNALS

ACTIVE IMPLANTABLE MEDICAL DEVICE WITH MEANS FOR TESTING VENTRICULAR CAPTURE BY ANALYSING AN ENDOCARDIAC ACCELERATION SIGNAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2010 FR 1056098**

(43) Date de publication de la demande:
**01.02.2012 Bulletin 2012/05**

(73) Titulaire: **Sorin CRM SAS**
**92143 Clamart Cedex (FR)**

(72) Inventeurs:
• **Giorgis, Lionel**
**22000, Saint-Brieuc (FR)**
• **Amblard, Amel**
**92290, CHATENAY MALABRY (FR)**

(74) Mandataire: **Grünecker Patent- und Rechtsanwälte**
**PartG mbB**
**Leopoldstraße 4**
**80802 München (DE)**

(56) Documents cités:
**EP-A1- 2 105 843       EP-A2- 1 995 685**
**US-A1- 2008 021 336    US-A1- 2009 281 590**
**US-A1- 2010 023 082**

**Description**

L'invention concerne les dispositifs médicaux implantables actifs tels que définis par la directive 90/385/CEE du 20 juin 1990 du Conseil des communautés européennes, plus précisément les implants permettant de surveiller en continu le rythme cardiaque et délivrer si nécessaire au cœur des impulsions électriques de stimulation, de resynchronisation et/ou de défibrillation en cas de trouble du rythme détecté par le dispositif.

Ces dispositifs comprennent des moyens de stimulation ventriculaire, aptes à délivrer des impulsions de faible énergie à diverses électrodes implantées dans le ventricule droit et/ou le ventricule gauche.

Après la délivrance de l'impulsion, il est important de recueillir l'onde évoquée, c'est-à-dire l'onde de dépolarisation induite par la stimulation du ventricule, afin de déterminer si cette stimulation a été efficace ou non. Ce test, dit "test de capture" est notamment utilisé pour évaluer le seuil d'entraînement, c'est-à-dire le niveau minimal d'énergie de stimulation nécessaire pour provoquer la dépolarisation du ventricule. Il convient en effet d'ajuster l'amplitude et/ou la largeur de l'impulsion de stimulation afin, d'une part, de garantir que toute stimulation provoquera une onde évoquée et que, d'autre part, l'énergie délivrée ne sera pas excessive pour ne pas trop obérer la durée de vie de l'implant du fait d'une consommation excessive.

Le test de capture est également un aspect important pour la surveillance du fonctionnement des dispositifs dits "CRT" (*Cardiac Resynchronisation Therapy*), qui sont des dispositifs munis d'électrodes permettant de stimuler l'un et l'autre ventricule. Le dispositif est capable de surveiller le rythme cardiaque et délivrer si besoin des impulsions permettant de stimuler conjointement les ventricules gauche et droit afin de resynchroniser ces derniers.

Ces stimulations respectives sont appliquées avec un délai interventriculaire variable, positif ou négatif, ajusté de manière à resynchroniser la contraction des ventricules et optimiser l'état hémodynamique du patient. Le test de capture doit permettre de s'assurer que la stimulation est bien effective sur l'un et l'autre des deux ventricules, car il s'agit là d'une condition essentielle de la resynchronisation de ces derniers. Or on constate généralement des seuils d'entraînement différents entre le site droit et le site gauche, ce qui peut conduire à une stimulation défectueuse. Le dispositif peut en outre opérer une détection erronée des ondes de dépolarisation par confusion entre une dépolarisation électrostimulée à l'endroit du site analysé, et une dépolarisation en provenance du site voisin captée indirectement.

Enfin, toute modification du délai interventriculaire peut avoir pour conséquence une modification des paramètres de la capture, de sorte qu'il est indispensable de suivre de manière très précise le fonctionnement du dispositif à chaque ajustement de ce paramètre, pour s'assurer que la thérapie de resynchronisation sera toujours efficace.

Il existe de nombreuses techniques pour procéder au test de capture ventriculaire.

Les techniques usuelles, décrites par exemple dans les WO 93/02741 A1 (ELA Médical) et EP 0 935 979 A1 (ELA Médical), sont basées sur l'analyse des signaux électriques recueillis par le dispositif.

Plus récemment, il a été proposé de tester la capture à partir d'un signal délivré par un capteur détectant directement la contraction mécanique du myocarde, ce qui permet d'obtenir immédiatement une information sur la réponse du ventricule à la stimulation, en faisant abstraction des périodes de *blanking* et autres limitations inhérentes au recueil d'un signal électrique. En d'autres termes, il s'agit d'utiliser un signal fonctionnel représentatif de la mécanique cardiaque, à la place d'un signal issu de la propagation électrique de l'onde de dépolarisation.

Ainsi, le WO 2005/089866 A1 (Medtronic) propose d'utiliser dans un dispositif CRT un capteur de contraction mécanique à diverses fins telles que : l'optimisation du délai atrioventriculaire DAV dans le cas d'une stimulation double chambre, l'optimisation du délai interventriculaire DVV dans le cas d'une stimulation biventriculaire pour une thérapie de resynchronisation CRT, ainsi que, subsidiairement, la discrimination entre capture et perte de capture dans une ou plusieurs cavités cardiaques pendant la délivrance d'une thérapie de stimulation, mais sans autre détail.

Le US 2004/0260351 A1 (Holmstrom et al.) propose, quant à lui, un détecteur de réponse évoquée pour un dispositif à stimulation biventriculaire, où un signal de bioimpédance cardiaque (ou, en variante, un signal d'accélération) est soumis à une analyse morphologique permettant de discriminer entre capture effective et perte de capture.

Pour cela, le dispositif mesure (par une méthode des moindres carrés appliquée aux échantillons successivement recueillis sur une fenêtre temporelle prédéterminée) les distances euclidiennes entre, d'une part, le signal d'impédance recueilli après chaque double stimulation et, d'autre part, cinq *templates* (gabarits) de référence correspondant chacun à l'une des cinq situations prédéterminées suivantes : capture sur les deux ventricules ; capture sur le ventricule gauche seulement ; capture sur le ventricule droit seulement ; perte de capture sur les deux ventricules ; conduction intrinsèque en l'absence de stimulation. Les cinq distances ainsi évaluées sont comparées à des seuils de distance respectifs, pour décider de la classification des captures/pertes de capture.

Cette technique présente l'inconvénient de ce que la décision sur l'existence et la configuration des captures est prise en référence à une distance, valeur qui doit être relativisée et adaptée (à partir de quelle valeur considère-t-on que le signal est conforme à l'un des gabarits ?), les seuils auxquels sont comparées les distances euclidiennes étant des paramètres fortement patient-dépendants.

Un autre inconvénient de cette technique tient au fait que, comme cela arrive souvent dans le domaine biomédical, les signaux comparés deux à deux (signal recueilli vs. gabarits) ne sont pas des signaux synchrones. De ce fait, si l'on calcule une distance entre deux signaux décalés, même d'une faible valeur (par exemple de 5 ms), un biais très important peut être introduit dans le calcul de la distance, biais qui reste présent quand bien même les deux signaux seraient absolument identiques, et qui faussera donc la mesure et donc le résultat du test de capture.

Le EP 1 995 685 A2 (Biotronik CRM) décrit une méthode de même nature, où la capture est détectée à partir de *templates* prédéterminés et d'un calcul de similarité par calcul de distances.

Le US 2008/021336 A1 (Cardiosync) décrit une technique comparable de détection de capture, reposant sur l'extraction de signaux d'accélération endocardiaque permettant de résumer le *pattern* (signature) caractéristique du signal, avec ensuite détection de la capture éventuelle au moyen d'un unique paramètre dérivé de ce *pattern.*

L'un des buts est de proposer une nouvelle technique de détection de capture qui pallie ces inconvénients, notamment une technique :

- qui permette de prendre une décision sur une base qui ne soit pas patient-dépendante ;
- qui soit insensible aux décalages temporels, même importants, susceptibles d'affecter les signaux à comparer ;
- qui soit applicable indifféremment à la détection d'une capture sur un ventricule isolé (aussi bien droit que gauche) qu'à une détection de capture biventriculaire (pour un dispositif CRT) ; et
- qui puisse être implémentée avec des ressources logicielles relativement modestes, donc qui puisse être mise en œuvre au sein même du dispositif implanté et en temps réel.

La technique est basée sur l'analyse de l'accélération endocardiaque (ci-après désignée "EA"), qui est un paramètre qui reflète très précisément et en temps réel les phénomènes concourant au fonctionnement mécanique du myocarde et qui peut être mesuré par un accéléromètre couplé au muscle cardiaque, comme cela est décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée au fond du ventricule et intégrant un microaccéléromètre permettant de mesurer l'accélération endocardiaque.

On notera toutefois que, bien que dans la présente description on fasse principalement référence à l'analyse d'un signal EA délivré par un capteur placé sur une sonde endocavitaire, la technique est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boîtier d'un implant. La technique est également applicable à l'analyse d'un signal EA externe recueilli de manière non invasive, par exemple issu d'un capteur fixé sur la poitrine du patient au niveau du sternum.

Le signal d'accélération endocardiaque EA recueilli au cours d'un cycle cardiaque donné (ci-après "signal EA") forme deux composantes princiles (ci-après "composantes EA"), correspondant aux deux bruits majeurs du cœur (sons S1 et S2 du phonocardiogramme) qu'il est possible de reconnaître dans chaque cycle cardiaque :

- la première composante d'accélération endocardiaque ("composante EA1"), dont les variations d'amplitude sont étroitement liées aux variations de la pression dans le ventricule (l'amplitude maximale crête-à-crête, appelée "PEA1", de cette composante EA1 étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ;
- la seconde composante d'accélération endocardiaque ("composante EA2") qui, quant à elle, survient pendant la phase de relaxation ventriculaire isovolumique. Cette seconde composante est principalement produite par la fermeture des valves aortiques et pulmonaires.

Plus précisément, l'invention propose un dispositif médical implantable actif comportant de manière en elle-même connue, par exemple d'après le US2008/021336 A1 précité, les éléments émeneés dans le préambule de la revendication 1. De façon caractéristique de l'invention, l'invention comprend les éléments énoncés dans la partie caratérisante de la revendication 1. les sous revendications visent des formes de realisation subsidiaires, avantageuses.

On va maintenant décrire un exemple de mise en œuvre de l'invention, en référence aux dessins annexés où les mêmes références numériques désignent d'une figure à l'autre des éléments identiques ou fonctionnellement semblables.

La Figure 1 montre les chronogrammes relevés au cours de trois cycles cardiaques successifs d'un signal d'accélération endocardiaque EA et d'un tracé d'électrocardiogramme ECG correspondant.

Les Figures 2a et 2b montrent un exemple d'une composante EA, respectivement en présence d'un rythme stimulé avec capture et dans le cas d'un rythme spontané.

La Figure 3 est un organigramme présentant sous forme de schéma par blocs les diverses étapes permettant d'obtenir une composante EA de référence, en présence d'une capture puis en rythme spontané.

Les Figures 4 et 5 illustrent la position, dans un espace bidimensionnel, des divers points de mesure relevés pour des situations de rythme stimulé et spontané, expliquant deux manières possibles de partitionner cet espace en deux classes définissant la capture ou l'absence de capture.

La Figure 6 illustre, toujours dans ce même espace à deux dimensions, l'étape de vérification de la distance séparant les classes précédemment définies.

La Figure 7 est un organigramme présentant sous forme de schéma par blocs les étapes successives du test de capture dans un dispositif selon la présente invention.

On va maintenant décrire un exemple de mise en œuvre de l'invention. En ce qui concerne ses aspects logiciels, l'invention peut être mise en œuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en œuvre les fonctions de l'invention qui seront décrites ci-dessous. L'adaptation de ces appareils à la mise en œuvre des fonctions de l'invention est à la portée de l'homme du métier, et elle ne sera pas décrite en détail.

*Principe de l'analyse d'une composante EA*

Comme on l'a indiqué plus haut, le signal d'accélération endocardiaque EA recueilli au cours d'un cycle cardiaque forme deux composantes principales, correspondant aux deux bruits majeurs du cœur (sons S1 et S2 du phonocardiogramme) qu'il est possible de reconnaître dans chaque cycle cardiaque :

- la composante EA1, dont les variations d'amplitude sont étroitement liées aux variations de la pression dans le ventricule (l'amplitude maximale crête-à-crête, appelée "PEA1", de cette composante EA1 étant plus précisément corrélée au maximum positif de la variation de pression dP/dt dans le ventricule gauche) ;
- la composante EA2 qui survient pendant la phase de relaxation ventriculaire isovolumique et qui est principalement produite par la fermeture des valves aortiques et pulmonaires.

Le signal EA contient parfois une ou deux autres composantes, appelées EA3 et EA4, correspondant aux sons S3 et S4 du phonocardiogramme.

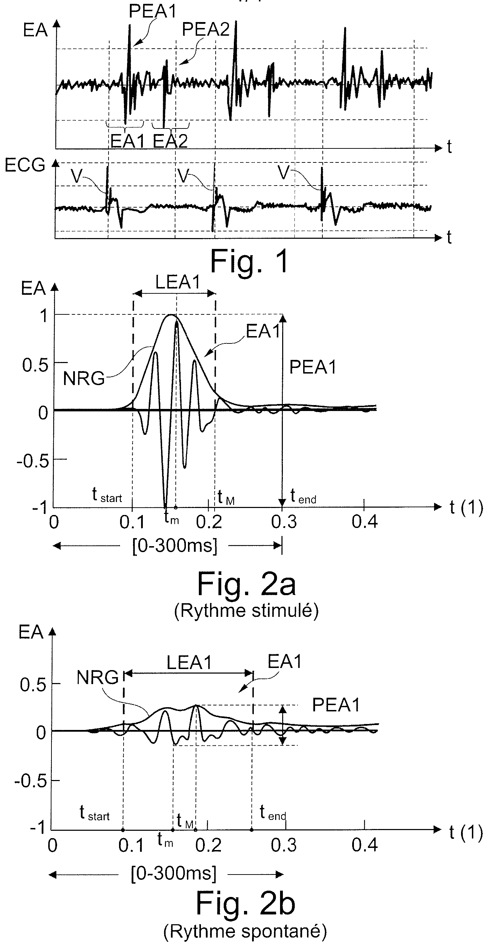

Ce signal EA est illustré Figure 1, qui montre les chronogrammes relevés au cours de trois cycles cardiaques successifs d'un signal d'accélération endocardiaque EA et d'un tracé d'électrocardiogramme ECG correspondant.

Le traitement préliminaire de la composante EA implique, tout d'abord, d'individualiser les cycles cardiaques successifs dans le signal EA recueilli en continu, en déterminant des marqueurs de début de cycle permettant de séparer ces cycles et d'isoler une série de sous-signaux EA bornés dans le temps correspondant chacun à une durée d'un seul cycle cardiaque :

- dans le cas d'un signal EA endocavitaire, les marqueurs temporels de début de cycle sont fournis par l'implant lui-même, qui selon le mode de fonctionnement garde en mémoire les instants de la stimulation V, (comme illustré Figure 1) ou bien les instants de détection de l'onde R ;
- dans le cas d'un signal EA externe, les marqueurs temporels de début de cycle cardiaque sont fournis par un algorithme de détection des pics de stimulation ou des complexes QRS du signal ECG, signal recueilli par ailleurs au moyen d'électrodes externes.

L'étape suivante consiste à isoler la composante EA1 et/ou la composante EA2 dans chacun des sous-signaux bornés dans le temps correspondant à un cycle cardiaque.

Dans la description qui va suivre, on s'intéressera principalement à la composante EA1, car c'est celle-ci qui est la plus caractéristique.

Mais les enseignements de l'invention sont transposables aussi bien à l'analyse de la composante EA2, en variante ou en complément de la composante EA1.

Chacune de ces composantes EA1 et EA2 sera représentée par un ensemble de valeurs successives décrivant la variation continue du signal EA dans une fenêtre temporelle donnée s'étendant autour du pic PEA1 (ou PEA2), sur une fraction de la durée d'un cycle cardiaque. Concrètement, chacune de ces composantes sera constituée d'un sous-ensemble de la matrice des N échantillons du signal EA obtenus après numérisation de celui-ci sur la durée du cycle cardiaque.

Chacune de ces composantes représente donc une fraction du signal EA sur la durée d'un cycle cardiaque, et chaque cycle cardiaque sera constitué d'une pluralité de composantes qui se succéderont, notamment les deux premières composantes EA1 et EA2, ces composantes étant également suivies des composantes secondaires EA3 et EA4.

De préférence, la composante EA1 (et/ou EA2) du signal EA est déterminée avec un moyennage sur plusieurs cycles, typiquement trois à cinq cycles, en appliquant une technique telle que celle exposée dans la demande EP 2 092 885 A1 (ELA Medical), technique qui permet notamment d'éliminer les variations cycle à cycle en recalant dans le temps les composantes successives avant de les moyenner.

Essentiellement, cette technique consiste à effectuer un prétraitement du signal EA recueilli en continu, avec :

- découpage du signal EA en sous-signaux correspondant chacun à la durée d'un cycle cardiaque et repérés par un marqueur de début de cycle permettant de réaliser ce découpage ;
- segmentation de chacun de ces sous-signaux de manière à individualiser une ou plusieurs des composantes EA1 et/ou EA2 dans une fenêtre temporelle donnée ;
- pour la composante courante EA1 ou EA2 ainsi isolée sur un cycle, recherche d'un pic d'intercorrélation par rapport aux composantes EA1 (ou EA2) des autres cycles recueillis ;
- calcul d'un décalage temporel correspondant ; et
- application du décalage temporel ainsi calculé à la composante courante, de manière à aligner celle-ci par rapport aux autres.

Les traitements d'analyse pourront être ensuite exécutés sur ces composantes EA1 (et/ou EA2) successives, avec élimination du biais de la variabilité cycle à cycle grâce à ce prétraitement.

*Définition des classes de discrimination entre capture et non-capture*

Cette phase initiale de la méthode vise à déterminer préalablement un critère de distinction permettant de reconnaître la présence ou l'absence d'une capture, par définition de deux classes correspondantes qui seront ensuite utilisées pour le test de capture proprement dit : selon que les valeurs courantes relevées au cours d'un cycle cardiaque appartiendront à l'une ou l'autre de ces deux classes, on considèrera qu'il y a présence ou absence d'une capture.

Ces classes sont définies par une partition d'un espace de dimension N en deux sous-espaces, l'un correspondant à une situation de capture, l'autre à une situation de non-capture.

Les N dimensions correspondent chacune à un indicateur caractéristique de la composante EA.

Pour la clarté et la simplicité de l'exposé, on ne fera référence comme "composante EA" qu'à la composante "EA1", mais tout ce qui sera dit à propos de cette composante "EA1" doit être considéré comme applicable *mutatis mutandis* à la composante EA2 et même, éventuellement, aux composantes EA3 et/ou EA4.

On entendra par "indicateur" une grandeur mesurable de la composante EA, obtenue par analyse du signal EA dans une fenêtre temporelle bornée correspondant à une fraction d'un cycle cardiaque comprenant cette composante EA. L'indicateur sera exprimé sous la forme d'une valeur mesurée unique, c'est-à-dire d'un scalaire. Chacun des indicateurs est choisi pour pouvoir caractériser par la valeur qu'il prend l'absence ou la présence d'une capture.

Concrètement, l'analyse d'un seul de ces indicateurs (par exemple la valeur PEA1 de l'amplitude crête-à-crête de la composante EA1) n'est pas suffisante pour discriminer de façon fiable entre présence et absence d'une capture, et conduirait à un nombre trop important de déterminations erronées, avec aussi bien des faux positifs que des faux négatifs.

C'est pourquoi l'invention propose d'utiliser une pluralité d'indicateurs différents (au moins deux), caractérisant une même composante EA recueillie au cours d'un battement cardiaque donné, et de combiner entre eux ces indicateurs, de la manière que l'on exposera, pour décider entre présence ou absence d'une capture avec un degré de fiabilité supérieur.

On appellera ci-après "vecteur" l'ensemble des valeurs de ces divers indicateurs obtenues pour une composante EA1 donnée d'un battement cardiaque (cette composante ayant été éventuellement moyennée sur plusieurs cycles successifs, comme on l'a exposé plus haut).

Dans l'exemple qui va suivre, on prendra l'exemple le plus simple, d'un vecteur de dimension N = 2, avec les deux indicateurs suivants, qui permettent de décrire simplement la morphologie de la composante EA1 d'une manière très différente selon qu'il y a ou non capture :

- la valeur PEA1 de *l'amplitude crête-à-crête* de la composante EA1 sur la fenêtre d'analyse considérée, par exemple sur la fenêtre [0-300 ms], l'origine des temps (0 ms) correspondant à l'instant de la stimulation ventriculaire la plus tardive, et
- la valeur LEA1 de la largeur de la composante EA1, c'est-à-dire *la durée* de celle-ci.

Ces indicateurs sont illustrés notamment Figures 2a et 2b, respectivement pour une composante EA1 obtenue en rythme stimulé avec capture, et pour une composante EA1 en rythme spontané.

En ce qui concerne plus précisément l'indicateur LEA1, celui-ci peut être obtenu en seuillant une enveloppe d'énergie NRG obtenue en élevant au carré la valeur des échantillons de signal, puis en appliquant une fenêtre de lissage de 100 ms par exemple. L'instant de début $t_{start}$ et l'instant de fin $t_{end}$ de début et de fin de la composante EA (avec LEA1 = $t_{end}$ - $t_{start}$) correspondent au franchissement d'un seuil qui peut être par exemple 10 % du maximum de l'énergie NRG sur la fenêtre [0-300 ms]. Cette méthode de détermination des instants caractéristiques de la composante EA1 est décrite, ainsi que d'autres, dans le EP 2 092 885 A1 précité, auquel on pourra se référer pour plus de détails.

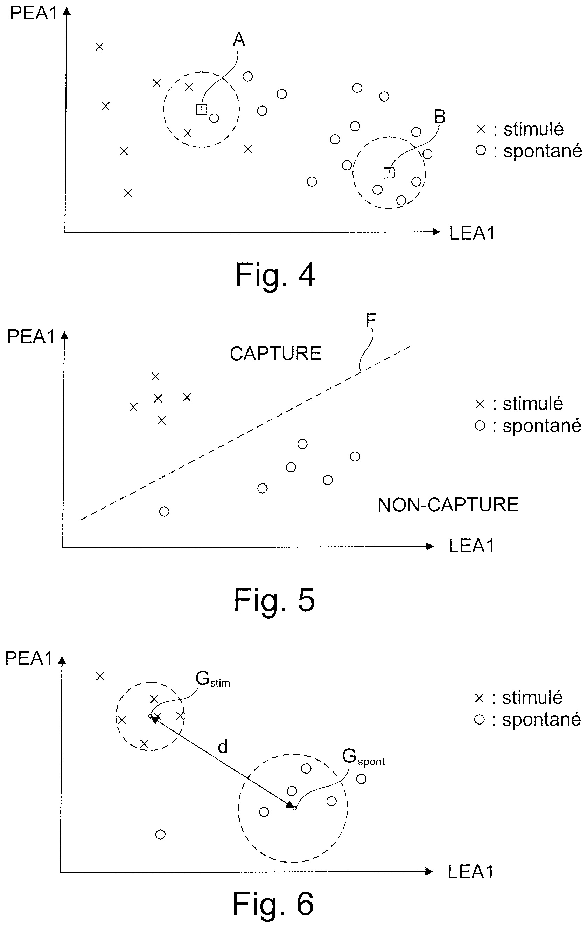

Chacun des couples de valeurs {PEA1, LEA1} constitue un vecteur représentatif de la composante EA1 analysée, vecteur qui peut être représentée graphiquement par un point dans le plan (PEA1, LEA1), comme illustré Figures 4 et 5.

L'exemple que l'on décrit avec N = 2 et où les indicateurs sont PEA1 et LEA1 n'est aucunement limitatif, et il est possible d'opérer l'analyse sur des vecteurs de dimension N > 2 incluant d'autres indicateurs, en variante ou en complément. Ainsi, parmi les indicateurs représentatifs, on peut utiliser notamment des indicateurs temporels tels que :

- l'*instant de survenue du pic* de la composante EA1, compté par rapport à l'origine de la fenêtre d'analyse [0-300 ms]. Cet instant peut être notamment calculé comme étant la moyenne des deux instants $t_m$ d'occurrence du pic minimum et $t_M$ du pic maximum du signal EA de la composante EA1 (cf. Figures 2a et 2b) ;
- les *instants de début et/ou de fin* $t_{start}$ et $t_{end}$, respectivement, de la composante EA1 (voir ci-dessus la manière dont ces instants sont définis) ;
- l'*instant du maximum de l'enveloppe énergétique* $t_{maxNRG}$ ; et
- les mêmes instants, pour la composante PEA2.

Il est également possible de choisir des indicateurs représentatifs de la morphologie de la composante EA1 tels que :

- le *rapport signal sur bruit* SNR, qui peut être défini par :

$$SNR\_EA1 = PEA1 / (2 \times \sigma\_noise),$$

$\sigma\_noise$ étant l'écart-type du signal considéré comme "bruit", c'est-à-dire le signal contenu dans la fenêtre de signal utile EA2, à l'exception du segment [$t_{EA2\_start}$, $t_{EA2-end}$] correspondant à la composante EA2 proprement dite ;
- la *valeur de contraste*, donnée par une formule telle que :

$$contraste\_EA1 = \frac{PEA1}{2 \cdot \sigma_{EA1\ window}}$$

$\sigma_{EA1\_window}$ étant l'écart-type du signal contenu dans la fenêtre [0, 300 ms] ;
- la *valeur d'entropie*, donnée par une formule telle que :

$$entropie\_EA1 = - \sum_{EA1\_window} (average\_ea\_cycles_i(t) \cdot \log10(average\_ea\_cycles_i(t)))$$

Cette quantité reflète le degré d'ordre du signal : si le signal est proche d'un bruit blanc l'entropie sera élevée, si au contraire il est ordonné l'entropie sera plus faible.
- l'*énergie cumulée*, calculée à partir de l'aire sous la courbe d'enveloppe énergétique NRG.

D'autres indicateurs caractéristiques peuvent être également utilisés en complément, tels que :

- les valeurs des amplitudes caractéristiques de la composante EA4, notamment l'amplitude crête-à-crête sur la fenêtre [- 200 ms, 0 ms].

On sait en effet que la composante EA4 arrive entre le début de l'activité auriculaire (l'onde P dans le cas d'un ECG, ou la détection de la dépolarisation sur la sonde auriculaire dans le cas d'un dispositif implantable) et le début de la composante EA1. On pourra se référer au EP 2 189 180 A1 (ELA Medical) pour plus de détails sur la manière de recueillir et analyser cette composante EA4.

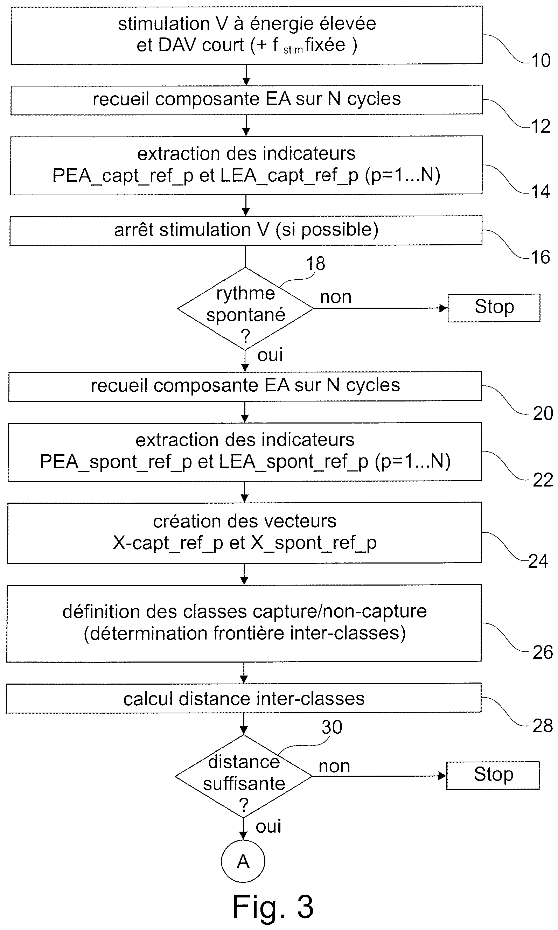

La Figure 3 illustre les diverses étapes de la phase initiale de la méthode décrite visant à définir deux classes de références (présence ou absence d'une capture), qui seront ensuite utilisées pour le test de capture proprement-dit.

Tout d'abord (bloc 10) le ventricule est stimulé dans des conditions permettant d'assurer la capture, avec une énergie de stimulation relativement élevée et un délai atrioventriculaire DAV court. Pour éviter les variations de rythme qui pourraient perturber le recueil de la composante EA, on peut également prévoir de stabiliser la fréquence cardiaque en stimulant le cœur par l'oreillette avec une fréquence fixe de stimulation auriculaire $f_{stim}$.

La composante EA1 (et/ou EA2) est ensuite recueillie et moyennée sur N cycles (bloc 12), typiquement N = 5 cycles successifs, de la manière expliquée plus haut.

Les indicateurs qui ont été choisis sont alors extraits de la composante EA (bloc 14). Dans l'exemple plus haut où ces indicateurs sont l'amplitude PEA du pic et la longueur LEA de la composante EA1, ces indicateurs extraits sur les N = 5 cycles successifs seront : *PEA_capt_ref_p* et *LEA_capt_ref_p* (*p* = 1... 5).

On arrête alors la stimulation du ventricule (bloc 16), bien entendu si cela est possible (absence de bloc atrioventriculaire complet, notamment).

On recherche ensuite (test 18) la présence d'un rythme spontané et, s'il est présent, on procède de la même façon que précédemment au recueil de la composante EA sur N = 5 cycles et à l'extraction des indicateurs correspondants *PEA_spont_ref_p* et *LEA_spont_ref_p* (blocs 20 et 22, semblables aux blocs 12 et 14).

On forme alors les *p* vecteurs *X_capt_ref_p* et les *p* vecteurs *X_spont_ref_p* à partir des indicateurs préalablement extraits (bloc 24).

L'étape suivante (bloc 26) consiste à trouver une règle de séparation des vecteurs *X_capt_ref_p* et *X_spont_ref_p* en deux classes distinctes.

Ces classes permettront ensuite, pour un nouveau vecteur X courant issu d'une nouvelle configuration de stimulation, de déterminer la présence ou l'absence d'une capture en fonction de la classification qui sera attribuée à ce nouveau vecteur courant.

Plusieurs méthodes peuvent être appliquées pour cette définition de la règle de séparation en deux classes.

Une première série de méthodes consiste à opérer séparément sur les deux indicateurs (c'est-à-dire sur les deux coordonnées du vecteur), par exemple au moyen de détecteurs simples à seuil, les seuils correspondant à chaque indicateur étant choisis pour obtenir un compromis sensibilité/sélectivité prédéterminé (par exemple : sensibilité > 90 % et spécificité > 95 %). Les deux tests sont ensuite combinés ensemble par un vote, par exemple une fonction ET logique.

Une autre série de méthodes, préférentiellement utilisées, consiste à opérer directement sur les vecteurs à deux dimensions (ou plus généralement à N dimensions, dans le cas de N > 2 indicateurs extraits de la composante EA).

Un vecteur donné peut être ainsi représenté graphiquement par un point de l'espace vectoriel, notamment par un point du plan (LEA1, PEA1) dans l'exemple donné ici, comme illustré Figures 4 et 5.

Une première méthode utilisable est la méthode dite "des K plus proches voisins" (KPPV), appliquée sur les vecteurs d'indicateurs, et illustrée Figure 4.

On calcule à cet effet les diverses distances entre le vecteur à tester (A ou B sur la Figure 4) et tous les vecteurs de la base d'apprentissage. On sélectionne alors les K vecteurs les plus proches du vecteur à tester (dans l'exemple illustré, K = 3) et on affecte au vecteur à tester la classe majoritaire, la répartition se faisant entre deux classes. Ainsi, sur l'exemple illustré, pour le vecteur A où deux des trois plus proches voisins font partie de la classe "stimulé" (X), le vecteur A sera considéré comme appartenant à cette classe "stimulé" tandis que pour le vecteur B dont les trois plus proches voisins font tous partie de la classe "spontané" (O) ce vecteur B sera considéré comme appartenant à la classe "spontané".

Une deuxième méthode utilisable consiste à mettre en œuvre un réseau de neurones, appliqué au vecteur d'indicateurs. Après une phase d'apprentissage permettant d'ajuster les paramètres internes du réseau de neurones, chaque nouveau vecteur testé traité par le réseau de neurones se verra affecter en sortie une classe d'appartenance parmi les deux (dans le présent exemple) classes possibles. On pourra utiliser comme réseau de neurones un réseau de type perceptron, qui est un réseau classifieur linéaire simple à n entrées et une seule sortie. On pourra trouver plus de détails dans : Simon Haykin, Neural Networks : A Comprehensive Foundation (2nd Edition), Prentice Hall, 1998 ou dans : Christopher M. Bishop, Neural Networks for Pattern Recognition, Oxford University Press, 1995.

Une troisième méthode utilisable consiste à opérer une classification linéaire par estimation de la matrice pseudo-inverse. On définit le vecteur *Classe* prenant les valeurs (1, -1) si X appartient à la classe *Capt_ref* et (-1, 1) si X appartient à la classe *Spont_ref.* L'apprentissage consiste à calculer la matrice W qui permet de minimiser l'erreur quadratique moyenne suivante :

$$W = \arg\min_{W} \left( \sum_{base\_apprentissage} \left( W \cdot \overset{r}{X_i} - Classe_i \right)^2 \right)$$

Les vecteurs $X_i$ de la base d'apprentissage (de classe $Classe_i$) sont les vecteurs $X\_spont\_ref\_p$ et $X\_capt\_ref\_p$. Cette matrice W est donné par l'expression suivante :

$$W = [Classe] \times [X]^T \times \left( [X] \times [X]^T \right)^{-1}$$

[x] étant la matrice résultant de la concaténation des vecteurs $X_i$ :

$$[X] = \left[ X\_capture\_REF_1, X\_capture\_REF_2, .., X\_spont\_REF_1, ... \right]$$

et [*Classe*] étant la matrice résultant de la concaténation des vecteurs $Classe_i$ associés aux vecteurs $X_i$.

Pour déterminer la classe d'un vecteur à tester Y, il suffit de calculer le vecteur $S = W \times Y = (S1, S2)$. Chaque composante de ce vecteur de sortie est une valeur (comprise entre -1 et 1) que le classifieur accorde à chaque classe. Il suffit d'affecter à Y la classe associée à la valeur maximale (si c'est S1, Y appartient à la classe *Capt_ref*).

La Figure 5 illustre le cas, simple, de l'espace (LEA1, PEA1) divisé en deux demi-espaces par une frontière F qui est une droite définissant la limite entre les deux zones capture/non-capture.

Mais selon l'approche retenue, la limite interzone peut être également formée par des segments de droite, un cercle, une parabole, ou toute autre forme géométrique. Cette limite peut aussi se traduire par une condition logique, par exemple dans le cas des KPPV, la condition d'appartenance d'un point X à une zone se déterminera en regardant la zone d'appartenance majoritaire des K points les plus proches de X.

On peut aussi proposer à un utilisateur d'ajuster manuellement la limite, en la traçant ou en la déplaçant sur un graphe (pas obligatoirement une droite), ou en entrant les coordonnées de deux points appartenant à la limite, si cette limite est une droite.

Avantageusement, une fois les deux classes déterminées, il est prévu une étape de vérification de la distance inter-classes (bloc 28 sur la Figure 3), de manière à s'assurer du caractère réellement discriminant de la classification que l'on vient d'établir.

La distance inter-classes est définie comme la distance euclidienne entre les centres de gravité des deux zones capture/non-capture, et l'on vérifie que cette distance est suffisamment grande par rapport aux distances moyennes entre les éléments de chaque zone par rapport aux centres de gravité respectifs.

Ceci est illustré notamment Figure 6, où d représente la distance séparant le centre de gravité $G_{stim}$ des vecteurs classés dans la zone "capture" du centre de gravité $G_{spont}$ des vecteurs classés dans la zone "non-capture". Cette vérification peut être opérée simplement en fixant un seuil pour décider que les deux zones sont suffisamment séparées.

Un autre critère consiste à calculer un ratio J, inspiré du critère discriminant de Fisher, constitué par le rapport entre la "distance entre les barycentres" ($m_1$ et $m_2$) et la "compacité des classes" ($s_1^2 + s_2^2$) :

$$J = \frac{|m_1 - m_2|^2}{s_1^2 + s_2^2} \text{, avec } m_i = \frac{1}{N} \sum_{Classe\_i} X_p \text{ et } s_i = \sum_{Classe\_i} \left( X_p - m_i \right)^2$$

Ce critère augmente avec :

- la distance entre les projections des barycentres des classes, et avec
- la "compacité" des projections des classes.

Si le critère de distance suffisante inter-classes n'est pas vérifié, ceci signifie qu'il n'y a pas ou peu de différence entre le signal EA recueilli en stimulant le ventricule et le signal EA recueilli en rythme spontané. Cette situation peut être aussi la conséquence d'un problème lié à la sonde. Dans un tel cas, un test de capture n'aurait pas de sens, et il est mis fin au processus, avec éventuellement déclenchement d'une alarme.

Dans le cas contraire, c'est-à-dire si les indicateurs relevés pour les signaux EA respectivement en stimulé et en spontané sont suffisamment différents, on peut procéder au test de capture, que l'on va maintenant décrire.

*Test de capture et ajustement du niveau de stimulation*

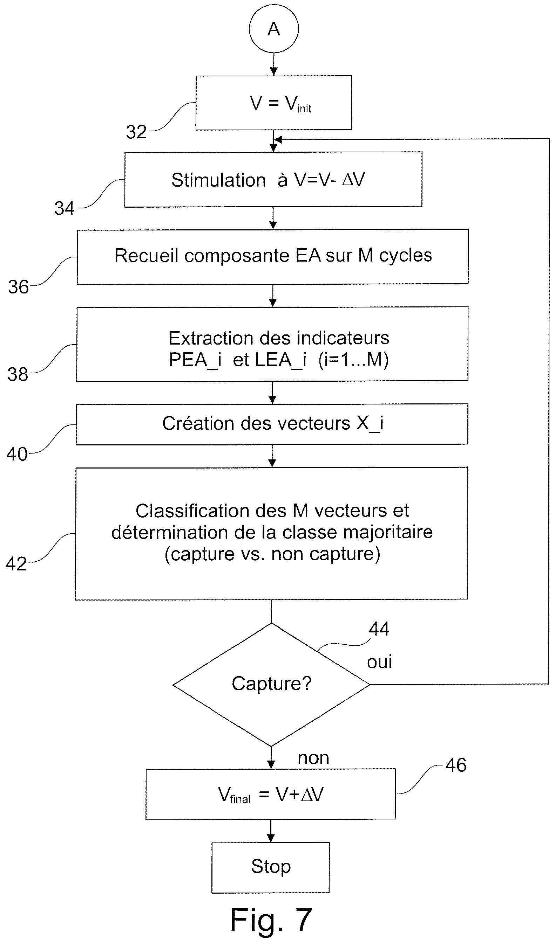

Les différentes étapes de la phase de test de capture sont illustrées Figure 7.

On va décrire ce test de capture ventriculaire dans le cadre d'une recherche du seuil d'entraînement (seuil de stimulation ventriculaire). Cette recherche consiste à appliquer au ventricule des impulsions de stimulation à énergie V décroissante, en contrôlant à chaque fois sur le signal EA la présence ou non d'une contraction par la méthode que l'on va décrire.

Si la contraction est effectivement présente, le dispositif considère que la stimulation ventriculaire est une stimulation efficace (il y a eu capture). L'énergie appliquée pour la stimulation ventriculaire suivante sera réduite, typiquement d'un pas d'amplitude fixe, par exemple $\Delta V = 0{,}25$ V. Lorsqu'une perte de capture sera détectée, le dispositif considérera que la dernière stimulation est inefficace, et donc que le seuil d'entraînement ventriculaire est supérieur à la dernière valeur appliquée. Dans ce dernier cas, une stimulation de sécurité à amplitude supérieure pourra être appliquée afin de provoquer une contraction ventriculaire.

Le seuil de stimulation ventriculaire ainsi déterminé peut être conservé dans la mémoire de l'appareil, être transmis à un centre de recueil de données, ou encore utilisé par l'implant pour modifier l'amplitude de stimulation appliquée au ventricule.

Pour d'autres détails sur les algorithmes d'ajustement de l'énergie de stimulation à partir de tests de capture successifs, on pourra se référer notamment au EP 1 080 744 A1 (ELA Medical), qui décrit diverses techniques de mesure du seuil, de contrôle de cohérence des mesures et d'ajustement de la largeur et de l'amplitude de l'impulsion de stimulation. Les algorithmes décrits dans ce document peuvent être mis en œuvre en utilisant comme test de capture celui de l'invention, par analyse de la morphologie du signal EA en lieu et place d'une détection électrique de la dépolarisation du ventricule.

Plus précisément, si l'on revient à la figure 7, le niveau de l'énergie de stimulation est fixé à une valeur initiale $V = V_{init}$ (bloc 32), à partir de laquelle l'amplitude de stimulation va être réduite de manière itérative (étape 34) par pas $\Delta V$.

La composante EA (EA1 et/ou EA2) est ensuite recueillie (bloc 36) de la manière exposée plus haut, puis les indicateurs sélectionnés correspondant à cette composante en sont extraits (bloc 38), sur M cycles, dans l'exemple illustré les indicateurs *PEA_i* et *LEA_i*, avec $i = 1...M$.

Les vecteurs correspondants *X_i* sont créés à partir des 9 indicateurs ainsi extraits (bloc 40). On classifie ensuite ces M vecteurs (bloc 42) selon l'une des méthodes exposées plus haut (KPPV, réseau de neurones, estimation de la matrice pseudo-inverse, etc.), et l'on choisit d'affecter la valeur d'amplitude de stimulation V à la classe majoritaire parmi les M.

On choisit pour M de préférence une valeur impaire, par exemple M = 3, mais l'on peut également dans une version simplifiée ne considérer qu'un seul couple d'indicateurs *PEA_1* et *LEA_1*, de manière à réduire le temps de réponse de l'algorithme.

En fonction de la classe majoritaire ainsi déterminée, on considère qu'il y a ou qu'il n'y a pas capture (test 44) :

- s'il y a eu perte de capture, la stimulation est alors rétablie à sa valeur antérieure de niveau d'énergie (bloc 46) et le processus est considéré comme achevé, dans la mesure où l'on considère que ce niveau de stimulation est le plus faible niveau permettant d'assurer la capture ;
- si la capture est présente, on revient alors à l'étape 34, pour diminuer encore le seuil de stimulation d'un incrément supplémentaire *ΔV* et tester à nouveau si, pour ce niveau de stimulation, la capture est encore présente, et ainsi de suite jusqu'à détecter la perte de la capture.

On notera que pour éviter les effets des variations du rythme, il est possible de faire le test de capture avec une fréquence de stimulation fixée résultant d'une stimulation auriculaire contrôlée, de la même manière que lors de la détermination de la frontière entre les deux classes.

*Application à un dispositif à stimulation biventriculaire (dispositif CRT)*

La stimulation biventriculaire implique bien entendu un test de capture effectué sur l'un et l'autre des ventricules droit et gauche.

En outre, pour l'optimisation de la thérapie de resynchronisation, la stimulation biventriculaire impose de réajuster le délai interventriculaire DVV à intervalles réguliers. Ce réajustement du DVV est souvent réalisé par balayage du délai entre un DVV minimum et un DVV maximum, afin de rechercher le maximum d'un paramètre hémodynamique au cours de ce balayage, maximum correspondant à l'optimum du DVV. La modification peut être également réalisée par un simple changement de valeur du DVV.

Dans tous les cas, le changement du DVV peut avoir une incidence sur la capture, par exemple si l'on stimule avec un DVV important, une dépolarisation spontanée du ventricule stimulé en second peut survenir avant délivrance de la stimulation à ce ventricule. Le test de capture doit donc être réitéré lors de chaque modification du DVV.

## Revendications

1. Un dispositif médical implantable actif du type prothèse cardiaque de stimulation, resynchronisation et/ou défibrillation, comportant :

- des moyens de stimulation ventriculaire, aptes à délivrer des impulsions de stimulation de faible énergie destinées à être appliquées à une électrode implantée dans la cavité ventriculaire, droite et/ou gauche, d'un patient,

les moyens de stimulation étant des moyens de stimulation conjointe des deux ventricules, droit et gauche avec application d'un délai interventriculaire entre les instants respectifs de stimulation des ventricules droit et gauche ;
- un capteur d'accélération, apte à délivrer un signal EA d'accélération endocardiaque
des moyens d'analyse du signal EA délivré par le capteur, comprenant des moyens aptes à:

isoler dans le signal EA au moins une composante EA d'accélération endocardiaque correspondant au premier (EA1) et/ou au second (EA2) bruit majeur du cœur, cette composante EA décrivant la variation continue du signal EA dans une fenêtre temporelle bornée correspondant à une fraction d'un cycle cardiaque ;
extraire de la composante EA ainsi isolée n indicateurs (PEA_i, LEA_i), avec n>=2, représentatifs du signal EA ; et
former un vecteur EA (X_i) de dimension n à partir des indicateurs ainsi extraits ; et

- des moyens de test de capture ventriculaire, aptes à détecter la survenue d'une contraction du ventricule consécutive à l'application d'une impulsion de stimulation et à déterminer en réponse la présence ou bien l'absence d'une capture ventriculaire, les moyens de test de capture étant des moyens aptes à détecter la présence d'un capture sur les deux ventricules et le dispositif comportant des moyens pour activer les moyens de test de capture en réponse à toute variation ou modification du délai interventriculaire ;

ces moyens de test de capture ventriculaire opérant en réponse aux dits moyens d'analyse du signal EA ;
des moyens classificateurs aptes, au cours d'une phase préalable, à :

acquérir une pluralité de signaux EA à un niveau d'énergie de stimulation suffisamment élevé pour provoquer une capture et former une pluralité correspondante de premiers vecteurs EA de référence (X_capt_ref_p) ;
acquérir une pluralité de signaux EA en rythme spontané du patient en l'absence de stimulation ventriculaire et former une pluralité correspondante de seconds vecteurs EA de référence (X_spont_ref_p) ; et
à partir des premiers et seconds vecteurs ainsi formés, partitionner l'espace de dimension n des vecteurs EA en deux sous-espaces correspondant respectivement à la présence et à l'absence d'une capture,
dispositif caractérisé en ce queles moyens pour partitionner ledit espace de dimension n des vecteurs EA en deux sous-espaces comprennent des moyens de mise en œuvre d'un algorithme de classification linéaire par estimation de la matrice pseudo-inverse
dont l'apprentissage consiste à calculer la matrice W qui permet de minimiser l'erreur quadratique moyenne telle que :

$$W=[Classe] \times [X]^{T} \times ([X] \times [X]^{T})^{-1}$$

avec [Classe] la matrice résultant de la concaténation des vecteurs Classe$_i$ associés aux vecteurs Xi, le vecteur Classe prenant les valeurs (1,-1) si X appartient à la classe Capt_ref et (-1,1) si X appartient à la classe Spont_ref, et
[X] étant la matrice résultant de la concaténation des vecteurs Xi;

et en ce que lesdits moyens de test de capture ventriculaire comprennent des moyens adaptés à :

acquérir au moins un signal EA à un niveau courant d'énergie de stimulation et former au moins un vecteur EA courant (X_i) correspondant ; et

discriminer la présence ou l'absence d'une capture en fonction de la position du vecteur EA courant dans ledit espace de dimension n des vecteurs EA,

respectivement dans l'un ou l'autre desdits deux sous-espaces.

2. Le dispositif de la revendication 1, dans lequel les n indicateurs représentatifs du signal EA sont choisis parmi les indicateurs du groupe formé par : la valeur de l'amplitude crête-à-crête (PEA1), la largeur (LEA1), l'instant de survenue du pic ($t_m$ ,$t_M$), l'instant de début ($t_{start}$), et l'instant de fin ($t_{end}$) de la composante EA1.

3. Le dispositif de la revendication 2, dans lequel les n indicateurs représentatifs du signal EA comprennent en outre des indicateurs de morphologie choisis parmi les indicateurs du groupe formé par : le rapport signal sur bruit, la valeur de contraste, la valeur d'entropie, et l'énergie cumulée de la composante EA1.

4. Le dispositif de la revendication 2, dans lequel les n indicateurs représentatifs du signal EA comprennent en outre des indicateurs choisis parmi les indicateurs du groupe formé par : la valeur de l'amplitude crête-à-crête, la largeur, l'instant de survenue du pic, l'instant de début, et l'instant de fin de la composante EA2.

5. Le dispositif de la revendication 2, dans lequel les n indicateurs représentatifs du signal EA comprennent en outre : la valeur de l'amplitude crête-à-crête de la composante EA4.

6. Le dispositif de la revendication 1, dans lequel n = 2 et les deux indicateurs représentatifs du signal EA sont la valeur de l'amplitude crête-à-crête (PEA1) et la largeur (LEA1) de la composante EA1.

7. Le dispositif de la revendication 1, dans lequel les moyens de test de capture ventriculaire comprennent des moyens aptes à former une pluralité de vecteurs EA courants, et à discriminer la présence ou l'absence d'une capture par application d'un critère majoritaire.

8. Le dispositif de la revendication 1, dans lequel les moyens pour partitionner ledit espace de dimension n des vecteurs EA en deux sous-espaces comprennent des moyens pour appliquer un critère de seuil séparément à chacun des indicateurs des vecteurs EA.

9. Le dispositif de la revendication 1, dans lequel les moyens classificateurs comprennent en outre des moyens d'évaluation d'une distance inter-classes et de vérification d'une distance minimale.

10. Le dispositif de la revendication 1, comprenant en outre des moyens de stimulation auriculaire, activables à une fréquence de stimulation fixe prédéterminée lors de la mise en œuvre des moyens classificateurs et des moyens de test de capture ventriculaire.

11. Le dispositif de la revendication 1, comprenant en outre des moyens de recherche du seuil de capture ventriculaire, aptes à modifier de manière itérative le niveau d'énergie de l'impulsion de stimulation, et à tester à chaque fois la présence ou l'absence d'une capture.

12. Le dispositif de la revendication 1, dans lequel le capteur d'accélération est un capteur du groupe formé par : capteur endocavitaire ; capteur épicardique ; capteur externe.

**Patentansprüche**

1. Aktive implantierbare medizinische Vorrichtung vom Typ kardiale Prothese zum Stimulieren, Resynchronisieren und/oder Defibrillieren, wobei sie Folgendes aufweist:

- Mittel zur Kammerstimulierung, die dazu befähigt sind, Stimulationsimpulse von geringer Energie abzugeben, die dazu bestimmt sind, auf eine Elektrode einzuwirken, die im Kammerhohlraum, rechts und/oder links, eines Patienten implantiert ist, wobei es sich bei den Stimulierungsmitteln um Mittel zur gemeinsamen Stimulierung der beiden Kammern, rechts und links, handelt, wobei zwischen den Zeitpunkten, zu denen die rechte beziehungsweise die linke Kammer stimuliert wird, ein interventrikuläre Verzögerung zur Anwendung gebracht wird;
- einen Beschleunigungssensor, der dazu befähigt ist, ein EA-Signal der endokardialen Beschleunigung zu liefern;

Mittel zur Untersuchung des EA-Signals, welche von dem Sensor geliefert wird, wobei sie Mittel umfassen, welche zum Folgendem befähigt sind:

aus dem EA-Signal mindestens eine EA-Komponente der endokardialen Beschleunigung zu isolieren, welche dem ersten (EA1) und/oder dem zweiten (EA2) Hauptgeräusch des Herzens entspricht, wobei diese EA-Komponente die kontinuierliche Variation des EA-Signals in einem Zeitfenster beschreibt, welches einer Fraktion eines Herzzyklus entspricht;

aus der EA-Komponente, welche auf diese Weise isoliert wurde, n Kennwerte (PEA_i, LEA_i), mit n >= 2, zu extrahieren, die für das EA-Signal repräsentativ sind; und

ausgehend von den Kennwerten, welche auf diese Weise extrahiert wurden, einen Vektor EA (X_i) mit der Dimension n zu bilden; und

- Mittel zur Überprüfung des ventrikulären Reizansprechens, die dazu befähigt sind, das Eintreten einer Kontraktion der Kammer infolge des Einwirkens eines Stimulationsimpulses nachzuweisen und, als Antwort darauf, das Vorliegen oder aber Ausbleiben eines ventrikulären Reizansprechens zu bestimmen, wobei es sich bei den Mitteln zur Überprüfung des Reizansprechens um Mittel handelt, die dazu befähigt sind, bei den beiden Kammern das Vorliegen eines Reizansprechens nachzuweisen, und wobei die Vorrichtung Mittel aufweist, um die Mittel zur Überprüfung des Reizansprechens als Antwort auf jedwede Variation oder Modifizierung der interventrikulären Verzögerung zu aktivieren;

wobei die Mittel zur Überprüfung des ventrikulären Reizansprechens als Antwort auf die Mittel zur Untersuchung des EA-Signals betrieben werden;

Mittel zur Klassifizierung, die im Laufe einer vorgeschalteten Phase zu Folgendem befähigt sind:

eine Mehrzahl an EA-Signalen bei einem Niveau an Stimulierungsenergie zu erfassen, das ausreichend hoch ist, um ein Reizansprechen hervorzurufen, und eine entsprechende Mehrzahl an ersten Referenz-EA-Vektoren (X_capt_ref p) zu bilden;

bei dem Patienten eine Mehrzahl an EA-Signalen in spontanem Rhythmus zu erfassen, wenn keinerlei Kammerstimulierung erfolgt, und eine entsprechende Mehrzahl an zweiten Referenz-EA-Vektoren (X_spont_ref_p) zu bilden; und

ausgehend von den ersten und den zweiten Vektoren, welche auf diese Weise gebildet wurden, den Dimensionsraum n der EA-Vektoren in zwei Teilräume zu unterteilen, die dem Vorliegen beziehungsweise dem Ausbleiben eines Reizansprechens entsprechen,

wobei die Vorrichtung **dadurch gekennzeichnet ist, dass** die Mittel zum Unterteilen des Dimensionsraums n der EA-Vektoren in zwei Teilräume Mittel zum Umsetzen eines Algorithmus umfassen, welcher zur linearen Klassifizierung durch Schätzen der pseudo-inversen Matrix bestimmt ist,

wobei dessen Lernvorgang darin besteht, die Matrix W zu berechnen, mittels welcher der mittlere Quadratfehler derart minimiert werden kann, dass Folgendes gilt:

$$W=[Klasse] \times [X]^T \times ([X] \times [X]^T)^{-1}$$

wobei als [Klasse] die Matrix bezeichnet wird, welche sich infolge der Konkatenation der Vektoren Klasse_i in Verbindung mit den Xi-Vektoren ergibt, wobei der Vektor Klasse die Werte (1, -1), wenn X der Klasse Capt_ref angehört, und (-1, 1) annimmt, wenn X der Klasse Spont_ref angehört, und

wobei [X] die Matrix ist, welche sich infolge der Konkatenation der Xi-Vektoren ergibt;

und dadurch, dass die Mittel zur Überprüfung des ventrikulären Reizansprechens Mittel umfassen, welche zu Folgendem befähigt sind:

mindestens ein EA-Signal bei einem laufenden Niveau an Stimulierungsenergie zu erfassen und mindestens einen entsprechenden laufenden EA-Vektor (X_i) zu bilden; und

in Abhängigkeit von der Position des laufenden EA-Vektors in dem Dimensionsraum n der EA-Vektoren, nämlich in dem einen beziehungsweise dem anderen der beiden Teilräume, zwischen dem Vorliegen und dem Ausbleiben eines Reizansprechens zu unterscheiden.

2. Vorrichtung nach Anspruch 1, wobei die n Kennwerte, welche repräsentativ für das EA-Signal sind, aus den Kennwerten der Gruppe ausgewählt, welche aus den folgenden gebildet ist: dem Wert der Spitze-zu-Spitze-Amplitude (PEA1), der Breite (LEA1), dem Zeitpunkt des Eintretens des Spitzenwertes ($t_m$, $t_M$), dem Zeitpunkt des Beginns (tstart) und dem Zeitpunkt des Endes (t_end) der Komponente EA1.

3.  Vorrichtung nach Anspruch 2, wobei die n Kennwerte, welche repräsentativ für das EA-Signal sind, darüber hinaus Morphologie-Kennwerte umfassen, die aus der Gruppe ausgewählt sind, welche aus den folgenden gebildet ist: dem Signal-RauschVerhältnis, dem Kontrastwert, dem Entropiewert und der kumulierten Energie der Komponente EA1.

4.  Vorrichtung nach Anspruch 2, wobei die n Kennwerte, welche repräsentativ für das EA-Signal sind, darüber hinaus Kennwerte umfassen, welche aus den Kennwerten der Gruppe ausgewählt sind, welche aus den folgenden gebildet ist: dem Wert der Spitze-zu-Spitze-Amplitude, der Breite, dem Zeitpunkt des Eintretens des Spitzenwertes, dem Zeitpunkt des Beginns und dem Zeitpunkt des Endes der Komponente EA2.

5.  Vorrichtung nach Anspruch 2, wobei die n Kennwerte, welche repräsentativ für das EA-Signal sind, darüber hinaus Folgendes umfassen: den Wert der Spitze-zu-Spitze-Amplitude der Komponente EA4.

6.  Vorrichtung nach Anspruch 1, wobei n = 2 ist und es sich bei den beiden Kennwerten, welche repräsentativ für das EA-Signal sind, um den Wert der Spitze-zu-Spitze-Amplitude (PEA1) und die Breite (LEA1) der Komponente EA1 handelt.

7.  Vorrichtung nach Anspruch 1, wobei die Mittel zur Überprüfung des ventrikulären Reizansprechens Mittel umfassen, die dazu befähigt sind, eine Mehrzahl an laufenden EA-Vektoren zu bilden, und durch Anwendung eines mehrheitsbezogenen Kriteriums zwischen dem Vorliegen und dem Ausbleiben eines Reizansprechens zu unterscheiden.

8.  Vorrichtung nach Anspruch 1, wobei die Mittel zum Unterteilen des Dimensionsraum n der EA-Vektoren in zwei Teilräume Mittel umfassen, um ein Schwellenwert-Kriterium gesondert auf jeden der Kennwerte der EA-Vektoren anzuwenden.

9.  Vorrichtung nach Anspruch 1, wobei die Mittel zur Klassifizierung darüber hinaus Mittel zur Auswertung eines Abstands zwischen den Klassen und zur Überprüfung eines Mindestabstands umfassen.

10.  Vorrichtung nach Anspruch 1, wobei sie darüber hinaus zur Mittel zur Vorhofstimulierung umfasst, die mit einer festen Stimulationsfrequenz aktiviert werden können, welche bei der Inbetriebnahme der Mittel zur Klassifizierung und der Mittel zur Überprüfung des ventrikulären Reizansprechens vorbestimmt wird.

11.  Vorrichtung nach Anspruch 1, wobei sie darüber hinaus Mittel zur Suche nach dem Schwellenwert des ventrikulären Reizansprechens umfassen, die dazu befähigt sind, auf iterative Weise das Energieniveau des Stimulationsimpulses zu modifizieren und jedes Mal das Vorliegen oder Ausbleiben eines Reizansprechens zu überprüfen.

12.  Vorrichtung nach Anspruch 1, wobei es sich bei dem Beschleunigungssensor um einen Sensor der Gruppe handelt, die aus den folgenden gebildet ist: endokavitärer Sensor; epikardialer Sensor; externer Sensor.

**Claims**

1.  An active implantable medical device of the stimulation, resynchronisation and/or defibrillation cardiac prosthesis type, comprising:

    - ventricular stimulation means, capable of delivering low-energy stimulating pulses intended to be applied to an electrode implanted in the right and/or left ventricular cavity of a patient, the stimulation means being means for jointly stimulating the two right and left ventricles, with application of an interventricular delay between the respective right and left ventricle stimulation instants;
    - an acceleration sensor, capable of delivering an EA (endocardial acceleration) signal;

    means for analysing the EA signal delivered by the sensor, comprising means capable of:

    isolating in the EA signal at least one EA (endocardial acceleration) component corresponding to the first (EA1) and/or to the second (EA2) major sound of the heart, this EA component describing the continuous variation of the EA signal in a limited time window corresponding to a fraction of a cardiac cycle;

extracting from the EA component thus isolated n indicators (PEA_i, LEA_i), with n>=2, representative of the EA signal; and

forming a vector EA (X_i) of dimension n from indicators thus extracted; and

- ventricular capture test means, capable of detecting the occurrence of a contraction of the ventricle consecutive to the application of a stimulation pulse and to determine, in response, the presence or the absence of a ventricular capture, the capture test means being means capable of detecting the presence of a capture on the two ventricles and the device comprising means for activating the capture test means in response to any variation or modification of the interventricular delay; ventricular capture test means performing in response to said means for analysing the EA signal;

classifying means capable, during a prior phase, of:

acquiring a plurality of EA signals at a stimulation energy level, sufficiently high to lead to a capture and to form a corresponding plurality of first reference EA vectors (X_capt_ref p);

acquiring a plurality of EA signals in spontaneous rhythm of the patient in the absence of ventricular stimulation and form a corresponding plurality of second reference EA vectors (X_spont_ref_p);

and from first and second vectors thus formed, partition the dimension space n of the EA vectors into two spaces corresponding respectively to the presence and to the absence of a capture,

device **characterised in that** the means for partitioning said dimension space n of the EA vectors into two subspaces comprise means for implementing a linear classification algorithm by estimating the pseudo-inverse matrix the learning of which consists of calculating the matrix W which enables to minimise the mean square error such that:

$$W=[\text{Class}] \times [X]^T \times ([X] \times [X]^T)^{-1}$$

with [Class] the matrix resulting from the concatenation of the Class vectors, associated with the vectors Xi, the Class vector taking the values (1,-1) if X belongs to the class Capt_ref and (-1,1) if X belongs to the class Spont_ref, and

[X] being the matrix resulting from the concatenation of the vectors Xi;

and **in that** said ventricular capture test means comprise means adapted to:

acquiring at least one EA signal at a current level of stimulation energy and forming at least one corresponding current EA vector (X_i); and

distinguishing the presence or the absence of a capture according to the position of the current EA vector in said dimension space n of the EA vectors, respectively in either of said two subspaces.

2. The device of claim 1, wherein the n indicators representative of an EA signal are chosen from among the indicators of the group formed by: the value of the peak-to-peak amplitude (PEA1), the width (LEA1), the peak occurrence instant ($t_m$, $t_M$), the start instant (tstart), and the end instant ($t_{end}$) of the component EA1.

3. The device of claim 2, wherein the n indicators representative of the EA signal further comprise morphology indicators chosen from among the indicators of the group formed by: the signal/sound ratio, the contrast value, the entropy value, and the cumulated energy of the component EA1.

4. The device of claim 2, wherein the n indicators representative of the EA signal further comprise indicators chosen from among the indicators of the group formed by: the peak-to-peak amplitude value, the width, the peak occurrence instant, the start instant, and the end instant of the component EA2.

5. The device of claim 2, wherein the n indicators representative of the EA signal further comprise: the peak-to-peak amplitude value of the component EA4.

6. The device of claim 1, wherein n = 2 and the two indicators representative of the EA signal are the value of the peak-to-peak amplitude (PEA1) and the width (LEA1) of the component EA1.

7. The device of claim 1, wherein the ventricular capture test means comprise means capable of forming a plurality of current EA vectors, and of distinguishing the presence or the absence of a capture by application of a majority criterion.

8. The device of claim 1, wherein the means for partitioning said dimension space n of the EA vectors into two subspaces comprise means for applying a threshold criterion separately to each of the indicators of the EA vectors.

9. The device of claim 1, wherein the classifying means further comprise means for evaluating an inter-class distance and for verifying a minimum distance.

10. The device of claim 1, further comprising auricular stimulation means, which can be activated at a predetermined fixed stimulation frequency during the implementation of classifying means and of ventricular capture test means.

11. The device of claim 1, further comprising means for seeking the ventricular capture threshold, capable of iteratively modifying the energy level of the stimulation pulse, and to test, each time, the presence or the absence of a capture.

12. The device of claim 1, wherein the acceleration sensor is a sensor of the group formed by: endocavitary sensor; epicardial sensor; external sensor.

Fig. 1

Fig. 2a
(Rythme stimulé)

Fig. 2b
(Rythme spontané)

```
┌─────────────────────────────────────────┐
│  stimulation V à énergie élevée          │
│  et DAV court (+ f stim fixée )          │──── 10
└─────────────────────────────────────────┘
                    │
                    ▼
┌─────────────────────────────────────────┐
│  recueil composante EA sur N cycles      │──── 12
└─────────────────────────────────────────┘
                    │
                    ▼
┌─────────────────────────────────────────┐
│  extraction des indicateurs              │
│  PEA_capt_ref_p et LEA_capt_ref_p (p=1...N) │──── 14
└─────────────────────────────────────────┘
                    │
                    ▼
┌─────────────────────────────────────────┐
│  arrêt stimulation V (si possible)       │──── 16
└─────────────────────────────────────────┘
                    │
                    ▼
                   18
               ╱rythme╲        non      ┌──────┐
              ╱ spontané ╲─────────────▶│ Stop │
               ╲   ?    ╱               └──────┘
                ╲    ╱
                  │ oui
                  ▼
┌─────────────────────────────────────────┐
│  recueil composante EA sur N cycles      │──── 20
└─────────────────────────────────────────┘
                    │
                    ▼
┌─────────────────────────────────────────┐
│  extraction des indicateurs              │
│  PEA_spont_ref_p et LEA_spont_ref_p (p=1...N) │──── 22
└─────────────────────────────────────────┘
                    │
                    ▼
┌─────────────────────────────────────────┐
│  création des vecteurs                   │
│  X-capt_ref_p et X_spont_ref_p           │──── 24
└─────────────────────────────────────────┘
                    │
                    ▼
┌─────────────────────────────────────────┐
│  définition des classes capture/non-capture │
│  (détermination frontière inter-classes)  │──── 26
└─────────────────────────────────────────┘
                    │
                    ▼
┌─────────────────────────────────────────┐
│  calcul distance inter-classes           │──── 28
└─────────────────────────────────────────┘
                    │
                    ▼
                   30
              ╱distance ╲       non      ┌──────┐
             ╱ suffisante ╲────────────▶│ Stop │
              ╲    ?     ╱              └──────┘
               ╲      ╱
                 │ oui
                 ▼
                ╱─╲
               │ A │
                ╲─╱
```

# Fig. 3

Fig. 4

Fig. 5

Fig. 6

A

$V = V_{init}$

32

Stimulation à $V = V - \Delta V$

34

Recueil composante EA sur M cycles

36

Extraction des indicateurs
PEA_i et LEA_i (i=1...M)

38

Création des vecteurs X_i

40

Classification des M vecteurs et
détermination de la classe majoritaire
(capture vs. non capture)

42

Capture?

44

oui

non

$V_{final} = V + \Delta V$

46

Stop

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9302741 A1
- EP 0935979 A1
- WO 2005089866 A1
- US 20040260351 A1, Holmstrom
- EP 1995685 A2
- US 2008021336 A1
- EP 0515319 A1
- EP 2092885 A1
- EP 2189180 A1
- EP 1080744 A1

**Littérature non-brevet citée dans la description**

- **SIMON HAYKIN.** Neural Networks : A Comprehensive Foundation. Prentice Hall, 1998
- **CHRISTOPHER M. BISHOP.** Neural Networks for Pattern Recognition. Oxford University Press, 1995